# EUROPEAN PATENT SPECIFICATION

(11) **EP 3 820 585 B1**
(45) Date of publication and mention of the grant of the patent: **02.11.2022**
(21) Application number: 19737726.0
(22) Date of filing: 08.07.2019
(51) Int. Cl.: B01D 3/00, C07C 209/82, C07C 211/03, C12M 1/00, C12P 13/00

(54) **IMPROVEMENTS IN OR RELATING TO ORGANIC COMPOUNDS**
VERBESSERUNGEN AN ODER IM ZUSAMMENHANG MIT ORGANISCHEN VERBINDUNGEN
AMÉLIORATIONS DANS OU RELATIVES À DES COMPOSÉS ORGANIQUES

(30) Priority: 10.07.2018 US 201862696069 P
(43) Date of publication of application: 19.05.2021
(73) Proprietor: Givaudan SA, 1214 Vernier (CH)
(72) Inventor: CANNELL, Jonathon, Cincinnati, Ohio 45239 (US)
(74) Representative: Global Patents
(86) International application number: PCT/EP2019/068289
(87) International publication number: WO 2020/011725

(56) References cited:
- US-A1- 2009 325 245
- US-A1- 2012 132 379
- US-A1- 2015 284 315

## Description

### TECHNICAL FIELD

The present invention relates generally to a method for purifying ethanolamine from a fermentation composition.

### BACKGROUND

Ethanolamine (HO-CH₂-CH₂-NH₂) is used for a wide variety of applications, including as a raw material precursor for flavour products. Ethanolamine can also be used for the recovery and removal of acid gases from natural, fuel and process gas, for the production of monoalkanolamides for non-ionic detergents, emulsifiers and soaps, and for the synthesis of acelethanolamine, phenylethanolamine and 2-mercaptothiazole in the manufacture of various products.

Ethanolamine is currently produced on an industrial scale by reacting ethylene oxide with an excess of ammonia. However, this is not a natural process and therefore cannot be used to produce natural flavour components. WO 2007/144346 discloses a natural process for making ethanolamine involving fermentation of an ethanolamine-producing bacterium. However, this document does not disclose how the ethanolamine can then be purified from the fermented composition. Ethanolamine has a higher boiling point than water and acts as a non-volatile salt in a fermented composition such that other solvents (including solvents with higher boiling points than ethanolamine) will distil preferentially to ethanolamine. It is therefore desirable to provide new and/or improved methods for purifying ethanolamine from a fermented composition.

### SUMMARY OF THE INVENTION

In accordance with a first aspect of the present invention there is provided a method for purifying ethanolamine from a fermentation composition, the method comprising adding a base to the fermentation composition, and removing the ethanolamine from the fermentation composition by distillation. In accordance with the present invention there is alternatively provided a method for purifying ethanolamine from a fermentation composition, the method comprising adjusting the pH of the fermentation composition from about 8 or less to equal to or greater than about pH 9, and removing ethanolamine from the fermentation composition by distillation.

In accordance with a second aspect of the present invention there is provided a method for synthesising ethanolamine, the method comprising fermenting a carbon source in the presence of an ethanolamine-producing microorganism to produce a fermentation composition comprising ethanolamine, adding a base to the fermentation composition and/or adjusting the pH of the fermentation composition to equal to or greater than about pH 9, and removing ethanolamine from the fermentation composition by distillation.

In accordance with a third aspect of the present invention there is provided purified ethanolamine obtained by or obtainable by a method of the first or second aspect of the present invention.

In accordance with a fourth aspect of the present invention there is provided the various uses of ethanolamine obtained by or obtainable by a method of the first or second aspect of the present invention. For example, there is provided the use of ethanolamine obtained by or obtainable by a method of the first or second aspect of the present invention for the synthesis of flavour compounds or components.

A base is added to the fermentation composition to deprotonate at least some ethanolamine molecules in the fermentation composition. The amount of base added to the fermentation composition is an amount suitable to deprotonate at least some ethanolamine molecules in the fermentation composition and suitable to enable at least some ethanolamine in the fermentation composition to be removed by distillation. The amount of base added to the fermentation composition is an amount suitable to increase the amount of ethanolamine that can be removed by distillation compared to the amount of ethanolamine that can be removed by distillation without addition of the base to the fermentation composition. The amount of base added to the fermentation composition is an amount suitable to adjust the pH of the fermentation composition to equal to or greater than about pH 9.

US 2015/284315 describes a method of purifying 1,4-diaminobutane (putrescine) prepared by a fermentation process by raising the pH of the fermentation concentrate to at least 12. However, no such process is known for ethanolamine recovery.

In certain embodiments of any aspect of the present invention the pH of the fermentation composition is adjusted to about pH 10 or greater, for example about pH 11 or greater.

In certain embodiments of any aspect of the present invention a solvent that has a higher boiling point than ethanolamine is added to the fermentation composition prior to removing the ethanolamine by distillation. In certain embodiments, water in the fermentation composition is exchanged with the solvent that has a boiling point higher than ethanolamine. This may, for example, comprise adding the solvent to the fermentation composition and removing the water by distillation. The addition of the solvent or the solvent exchange may, for example, take place during or after adjustment of the pH of the fermentation composition to equal to or greater than about pH 9.

In certain embodiments of any aspect of the present invention the ethanolamine obtained by or obtainable by a method of the first or second aspect of the present invention has a purity equal to or greater than about 60 %.

In certain embodiments of any aspect of the present invention the method of the first or second aspect of the present invention has a yield of purified ethanolamine equal to or greater than about 60 % relative to the total amount of ethanolamine in the fermentation composition before distillation.

Certain embodiments of any aspect of the present invention may provide one or more of the following advantages:
- production of ethanolamine from a natural source;
- high or increased purity and/or yield of the ethanolamine product;
- precipitation of impurities (e.g. anionic impurities) in the fermentation composition;
- reduction in viscosity of a fermentation composition concentrate enabling a low or reduced temperature and/or pressure to be used for distillation;
- reduced degradation and/or reaction of ethanolamine resulting in increased yield;
- reduced salt and impurity concentration in fermentation composition resulting in reduced corrosion of metal vessels in downstream processes.

The details, examples and preferences provided in relation to any particulate one or more of the stated aspects of the present invention will be further described herein and apply equally to all aspects of the present invention.

### DETAILED DESCRIPTION

The present invention is based, at least in part, on the surprising finding that deprotonating ethanolamine increases its volatility and thus enables it to be separated from mixtures by distillation. It has been found that when ethanolamine is in its protonated form it behaves as a non-volatile salt in fermentation composition and cannot be distilled. It has further been found that other solvents (even solvents having a higher boiling point than ethanolamine) will be distilled preferentially to ethanolamine when it is in its protonated form. This has resulted in the method of the present invention, whereby ethanolamine is separated from a fermentation composition by adding a base and then removing ethanolamine from the fermentation composition by distillation. The present invention may further be based on the surprising finding that the addition of a base to a fermentation composition provides a counter-ion to impurities such as anion salts in the fermentation composition and thus precipitates the impurities in the fermentation composition. The precipitation can reduce solvent viscosity, allowing lower pressures and temperatures to be used for distillation. It may also prevent or reduce material from "bubbling" under heat and contaminating the distillate with impure feed material during a wiped film still distillation. The lower temperatures and pressures may then reduce degradation and/or reaction of the ethanolamine, thus increasing yield. The reduction of impurities in the fermentation composition may also reduce corrosion of metal vessels in which downstream processes are carried out.

Thus, there is provided herein a method for purifying ethanolamine from a fermentation composition, the method comprising adjusting the pH of the fermentation composition to equal to or greater than about pH 9, and removing ethanolamine from the fermentation composition by distillation. Herein, the method comprises adding a base to the fermentation composition, and removing ethanolamine from the fermentation composition by distillation. The ethanolamine is removed from the fermentation composition by distillation after the pH is adjusted to equal to or greater than about pH 9 after the base is added to the fermentation composition

By "purification" it is meant that the ethanolamine is separated from other substances in the fermentation composition, which may be referred to as contaminants or impurities. Thus, the amount of contaminants or impurities is reduced. 100 % pure ethanolamine comprises only ethanolamine, whereas 98 % ethanolamine comprises 98 % ethanolamine and 2 % other substances. This may be measured, for example, by NMR (purity given in mol%) or gas chromatography (purity given in total peak area %, TPA%). HPLC analysis may be used to give purity by % (wt/vol).

The ethanolamine product of the methods described herein may, for example, have a purity of at least about 60 % when measured by any one or more of the methods described above (e.g. 60 mol% when measured by NMR). For example, the ethanolamine products of the methods described herein may have a purity of at least about 65 %, for example at least about 70 %, for example at least about 75 %, for example at least about 80 %, for example at least about 85 %, for example at least about 90 %, for example at least about 95 %, for example at least about 96 %, for example at least about 97 %, for example at least about 98 %. For example, the ethanolamine products of the methods described herein may have a purity up to about 100 % or up to about 99 %. For example, the ethanolamine products may have a purity ranging from about 60 % to about 100 %, for example from about 75 % to about 100 %, for example from about 90 % to about 99 %.

The yield of purified ethanolamine relative to the total amount of ethanolamine present in the fermentation composition starting material (the total amount of ethanolamine present in the fermentation composition before distillation) may, for example, be equal to or greater than about 60 %. For example, the yield of purified ethanolamine may be equal to or greater than about 65 %, for example equal to or greater than about 70 %, for example equal to or greater than about 75 %, for example equal to or greater than about 80 %, for example equal to or greater than about 85 %, for example equal to or greater than about 90 %, for example equal to or greater than about 92 %, for example equal to or greater than about 94 %, for example equal to or greater than about 95 %. For example, the yield of purified ethanolamine may be up to about 100 %, for example up to about 99 %, for example up to about 98 %. For example, the yield of purified ethanolamine may range from about 60 % to about 100 %, for example from about 75 % to about 100 %, for example from about 80 % to about 100 %, for example from about 85 % to about 100 %, for example from about 90 % to about 99 %.

Yield of purified ethanolamine in the distillate relative to the amount of ethanolamine in the fermentation composition prior to distillation may be measured by calculating the total mass of ethanolamine in the final product (distillate) divided by the total mass of ethanolamine in the fermentation composition prior to distillation (but after fermentation is complete), multiplied by 100. If the distillate is within the specification >99% purity, the whole sample will be considered as ethanolamine. If the distillate is not within the specification of >99% purity, the total mass of ethanolamine in the distillate is adjusted accordingly (e.g. in the case of 85 wt% purity, the total mass of ethanolamine is multiplied by 0.85).

The method comprises purifying ethanolamine from a fermentation composition. In certain embodiments, the method also comprises making the fermentation composition comprising ethanolamine. The method may, for example, comprise synthesising ethanolamine by fermenting a carbon source in the presence of an ethanolamine-producing microorganism in order to provide a fermentation composition comprising ethanolamine. Alternatively, the fermentation composition may be obtained from a commercial source, for example from a commercial source that had made the fermentation composition by fermenting a carbon source in the presence of an ethanolamine-producing microorganism.

Ethanolamine may be synthesised by fermenting a carbon source in the presence of an ethanolamine-producing microorganism. A nitrogen source may also be provided for the synthesis of ethanolamine. The fermentation may occur at any temperature and pH suitable for the particular ethanolamine-producing microorganism that is used. For example, the fermentation may take place at a temperature ranging from about 20°C to about 55°C, for example from about 25°C to about 40°C. For example, the fermentation may take place at a pH in the range of about pH 6 to about pH 8, for example from about pH 6.5 to about pH 7.5, for example from about pH 6.8 to about pH 7. For example, ethanolamine may be synthesised by fermenting a carbon source in the presence of recombinant microorganisms, for example recombinant bacteria, that have been modified to express genes necessary to synthesise ethanolamine from the carbon source such as glucose. The recombinant microorganisms may have been modified to silence genes that result in the degradation and/or reaction of ethanolamine or its intermediates or that use ethanolamine or its intermediates in other pathways. Serine may, for example, be synthesised as an intermediate in the synthesis of ethanolamine. The serine may then be decarboxylated to ethanolamine, for example by a serine decarboxylase enzyme. Serine may, for example, be synthesised from 3-phosphoglycerate. Ethanolamine may, for example, be synthesised by one or more of the methods and/or using one or more of the microorganisms disclosed in WO 2007/144346, WO 2014/049382 and US20170211103.

The synthesis of ethanolamine as described herein results in a fermentation composition comprising ethanolamine. The methods described herein may, for example, further comprise a step of synthesising ethanolamine, for example as described herein.

By "fermentation composition" it is meant a liquid composition that is or is derived from a liquid composition suitable for culturing (maintaining the metabolic activity of, including growing and/or replicating) a microorganism. The fermentation composition may therefore comprise one or more nutrients required or useful for culturing a microorganism, in particular an ethanolamine-producing microorganism such as the ethanolamine-producing microorganism used to produce the ethanolamine present in the fermentation composition. The term "fermentation composition" encompasses filtrates and centrates (e.g. compositions where biomass such as cells, microorganisms and/or biological agents have been removed by, for example, filtration or centrifugation) and concentrates (e.g. compositions where water has been removed, for example where water has been replaced with a solvent having a higher boiling point than ethanolamine).

The fermentation composition used in the methods described herein comprises ethanolamine. The concentration of ethanolamine in the fermentation composition starting material may, for example, be at least about 20 g/L. For example, the concentration of ethanolamine in the fermentation composition may be at least about 25 g/L, for example at least about 30 g/L, for example at least about 35 g/L. For example, the concentration of ethanolamine in the fermentation composition may be up to about 100 g/L, for example up to about 90 g/L, for example up to about 85 g/L, for example up to about 80 g/L, for example up to about 75 g/L, for example up to about 60 g/L, for example up to about 50 g/L. For example, the concentration of ethanolamine in the fermentation composition starting material may be from about 20 g/L to about 100 g/L, for example from about 30 g/L to about 85 g/L, for example from about 35 g/L to about 75 g/L. This may refer to the concentration of ethanolamine in the fermentation composition after biomass has been removed.

The fermentation composition used in the methods described herein may or may not comprise an aqueous solvent. In particular, the fermentation composition may comprise an aqueous solvent whilst it is being used to culture the ethanolamine-producing microorganism used to produce the ethanolamine present in the fermentation composition and/or synthesise ethanolamine. The fermentation composition used in the methods described herein may or may not comprise a solvent having a higher boiling point than ethanolamine. The fermentation composition used in the methods described herein may or may not comprise biomass (organic material derived from plants, animals and microorganisms including cells, microorganisms and/or biological agents such as ethanolamine-producing microorganisms which may have been used to produce the ethanolamine present in the fermentation composition). Any biomass such as cells, microorganisms and/or biological agents present in the fermentation composition may be sterilized and/or removed from the fermentation composition prior to, during, or after the purification methods described herein.

The methods described herein may, for example, comprise sterilizing the fermentation composition prior to removing ethanolamine by distillation. By "sterilization" it is meant that any living cells and/or organisms and/or biological agents (including, for example, bacteria, viruses, fungi, prions and spores) are deactivated or killed. The fermentation composition may, for example, be sterilized by heating, irradiation, high pressure or adding chemicals such as ethylene oxide, nitrogen dioxide, ozone, glutaraldehyde, formaldehyde, hydrogen peroxide and peracetic acid.

Where the method comprises synthesising ethanolamine by fermenting a carbon source in the presence of an ethanolamine-producing microorganism, any sterilization occurs after ethanolamine has been produced. The sterilization may, for example, occur before, during or after the base is added and/or the pH of the fermentation composition is adjusted to equal to or greater than about pH 9, preferably before. The sterilization may, for example, occur before any cells, microorganisms and/or biological agents are removed from the fermentation composition. The sterilization may, for example, occur before, during or after any solvent having a higher boiling point than ethanolamine is added to the fermentation composition, preferably before. The sterilization may, for example, occur before, during or after any water in the fermentation composition is exchanged with a solvent having a higher boiling point than ethanolamine, preferably before.

The methods described herein may, for example, comprise removing biomass such as cells, microorganisms and/or biological agents from the fermentation composition prior to removing ethanolamine by distillation. Where the fermentation composition has been sterilized, the biomass such as cells, microorganisms and/or biological agents may be inactivated or dead. Removing biomass such as cells, microorganisms and/or biological agents from the fermentation composition may, for example, comprise filtration or centrifugation. The fermentation composition may, for example, be filtered through a filter having a pore size of 0.2 µm or less. The fermentation composition used in the presently described purification methods may, for example, be referred to as a filtrate and/or centrate where biomass such as cells, microorganisms and/or biological agents has been removed.

Where the method comprises synthesising ethanolamine by fermenting a carbon source in the presence of an ethanolamine-producing microorganism, any removal of biomass such as cells, microorganisms and/or biological agents occurs after ethanolamine has been produced. The removal of biomass such as cells, microorganisms and/or biological agents may, for example, occur before, during or after the base is added and/or the pH of the fermentation composition is adjusted to equal to or greater than about pH 9, preferably before. The removal of biomass such as cells, microorganisms and/or biological agents may, for example, occur after sterilization of the fermentation composition. The removal of biomass such as cells, microorganisms and/or biological agents may, for example, occur before, during or after any solvent having a higher boiling point than ethanolamine is added to the fermentation composition, preferably before.

The methods described herein may, for example, also comprise removal of any solid material in the fermentation composition (e.g. precipitates of impurities). This may, for example, occur after the base has been added and/or after the pH of the fermentation composition has been adjusted to equal to or greater than about pH 9.

The fermentation composition used in the methods described herein may have any pH suitable for culturing a microorganism, particularly an ethanolamine-producing microorganism used to produce the ethanolamine present in the fermentation composition. The fermentation composition may therefore typically have a pH in the range of about pH 6 to about pH 8, for example from about pH 6.5 to about pH 7.5, for example from about pH 6.8 to about pH 7. This refers to the pH of the fermentation composition before the base is added and the pH is adjusted to a pH equal to or greater than about 9 in accordance with the methods described herein (the term fermentation broth may be used to refer to the fermentation composition at the end of fermentation before further processing such as sterilization and/or removal of biomass). The methods described herein comprise adding a base to the fermentation composition and/or adjusting the pH of the fermentation composition to equal to or greater than about pH 9. The amount of base added to the fermentation composition may be any amount suitable to deprotonate at least some ethanolamine in the fermentation composition and/or enable at least some ethanolamine in the fermentation composition to be removed by distillation. The amount of base added to the fermentation composition may also be sufficient to deprotonate other species in the fermentation composition (such as amino acids and other byproducts of the fermentation process). This may alter the interaction of these other species with ethanolamine and consequently enable ethanolamine to be removed by distillation. The amount of base added to the fermentation composition is an amount suitable to increase the amount of ethanolamine that can be removed by distillation compared to the amount of ethanolamine removed by distillation without addition of the base. The amount of base added to the fermentation composition is an amount suitable to adjust the pH of the fermentation composition to equal to or greater than about pH 9. The precise amount of base required may vary depending on the strength of the base and/or the nature and composition of the fermentation composition.

The amount of base added to the fermentation composition may be an amount sufficient to deprotonate at least about 40 % of the ethanolamine molecules in the fermentation composition. For example, the amount of base added to the fermentation composition may be an amount sufficient to deprotonate at least about 45 %, for example at least about 50 %, for example at least about 55 %, for example at least about 60 %, for example at least about 65 %, for example at least about 70 %, for example at least about 75 %, for example at least about 80 %, for example at least about 85 %, for example at least about 90 %, for example at least about 95 % of the ethanolamine molecules. For example, the amount of base added to the fermentation composition may be an amount sufficient to deprotonate up to about 100 % of the ethanolamine molecules, for example up to about 98 % of the ethanolamine molecules. For example, the amount of base added to the fermentation composition may be an amount sufficient to deprotonate from about 40 % to about 100 %, for example from about 60 % to about 100 %, for example from about 70 % to about 100 %, for example from about 80 % to about 100 %, for example from about 90 % to about 100 %, for example from about 95 % to about 100 % of ethanolamine molecules in the fermentation composition. The pKa of ethanolamine (the pH at which 50 % of ethanolamine molecules are protonated) is 9.45.

By "base" it is meant a substance that accepts a proton (H⁺), in particular a substance that accepts a proton from ethanolamine. The base may, for example, be a strong base (a base that is completely dissociated in aqueous solution). The base may, for example, be a hydroxide base. The base may, for example, be selected from sodium hydroxide, lithium hydroxide, potassium hydroxide, rubidium hydroxide, cesium hydroxide, magnesium hydroxide, calcium hydroxide, strontium hydroxide, barium hydroxide or combinations of one or more thereof.

The methods described herein may comprise adjusting the pH of the fermentation composition to pH 9.5 or greater, for example pH 10 or greater, for example pH 10.5 or greater, for example pH 11 or greater, for example pH 11.1 or greater, for example pH 11.2 or greater, for example pH 11.3 or greater, for example pH 11.4 or greater. For example, the methods described herein may comprise adjusting the pH of the fermentation composition to about pH 11.45 or greater. The methods described herein may additionally comprise adjusting the pH of the fermentation composition to a pH equal to or less than about pH 14, for example equal to or less than about pH 13.5, for example equal to or less than about pH 13, for example equal to or less than about pH 12.5, for example equal to or less than about pH 12. For example, the methods described herein may comprise adjusting the pH of the fermentation composition to a pH in the range of from about pH 9 to about pH 14, for example about pH 10 to about pH 13, for example from about pH 11 to about pH 12.

The methods described herein further comprise removing ethanolamine from the fermentation composition by distillation. This takes place after the base has been added and the pH of the fermentation composition has been adjusted to equal to or greater than about pH 9.

By "distillation" it is meant a process for separating one substance from another by selective boiling and condensation. Any suitable method of distillation may be used. For example, wiped film still or short path still distillation may be used.

The distillation may, for example, occur at a vapour temperature equal to or less than about 175 °C. For example, the distillation may occur at a vapour temperature equal to or less than about 170°C or equal to or less than about 165 °C or equal to or less than about 160 °C or equal to or less than about 155 °C or equal to or less than about 150 °C or equal to or less than about 145 °C or equal to or less than about 140 °C or equal to or less than about 135 °C or equal to or less than about 130 °C or equal to or less than about 125°C or equal to or less than about 120°C.

The distillation may, for example, occur at a vapour temperature equal to or greater than about 55°C. For example, the distillation may occur at a vapour temperature equal to or greater than about 60°C or equal to or greater than about 65°C or equal to or greater than about 70°C or equal to or greater than about 75°C or equal to or greater than about 80°C or equal to or greater than about 85°C or equal to or greater than about 90°C or equal to or greater than about 95°C or equal to or greater than about 100°C.

The distillation may, for example, occur at a vapour temperature ranging from about 55°C to about 175°C, for example from about 75°C to about 150°C, for example from about 95 °C to about 140°C, for example from about 95°C to about 120°C.

The distillation may, for example, occur at a bottoms temperature equal to or less than about 300°C. For example, the distillation may occur at a bottoms temperature equal to or less than about 290°C or equal to or less than about 280°C or equal to or less than about 270°C or equal to or less than about 260°C or equal to or less than about 250°C or equal to or less than about 240°C or equal to or less than about 230°C or equal to or less than about 220°C or equal to or less than about 210°C or equal to or less than about 200°C.

The distillation may, for example, occur at a bottoms temperature equal to or greater than about 90°C. For example, the distillation may occur at a bottoms temperature equal to or greater than about 95°C or equal to or greater than about 98°C or equal to or greater than about 99°C or equal to or greater than about 100°C.

The distillation may, for example, occur at a bottoms temperature ranging from about 90°C to about 300°C, for example from about 95°C to about 280°C, for example from about 95°C to about 260°C, for example from about 95°C to about 250°C, for example from about 98°C to about 240°C, for example from about 99°C to about 220°C.

The distillation may, for example, occur at a pressure equal to or less than about 110 kPa. For example, the distillation may occur at a pressure equal to or less than about 101.3 kPa, for example equal to or less than about 80 kPa, for example equal to or less than about 60 kPa, for example equal to or less than about 50 kPa, for example equal to or less than about 40 kPa, for example equal to or less than about 20 kPa, for example equal to or less than about 10 kPa, for example equal to or less than about 5 kPa.

The distillation may, for example, occur at a pressure equal to or greater than about 0.05 kPa. For example, the distillation may occur at a pressure equal to or greater than about 0.1 kPa, for example equal to or greater than about 0.14 kPa, for example equal to or greater than about 0.2 kPa, for example equal to or greater than about 0.5 kPa.

The distillation may, for example, occur at a pressure ranging from about 0.05 kPa to about 110 kPa, for example from about 0.05 kPa to about 101.3 kPa, for example from about 0.1 kPa to about 101.3 kPa, for example from about 0.1 kPa to about 100 kPa.

The methods provided herein may, for example, comprise adding a solvent having a boiling point greater than the boiling point of ethanolamine to the fermentation composition. The methods provided herein may, for example, comprise exchanging any water present in the fermentation composition with a solvent having a boiling point greater than the boiling point of ethanolamine. This may, for example, comprise adding the solvent to the fermentation composition and removing the water by distillation. The solvent may, for example, be added to the fermentation composition, for example exchanged with water in the fermentation composition, before, during or after addition of the base and/or adjustment of the pH of the fermentation composition to a pH equal to or greater than about 9. In particular, where the pH of the fermentation composition is adjusted using an aqueous base, water in the fermentation composition may be exchanged with the solvent during or after addition of the base and/or adjustment of the pH of the fermentation composition to a pH equal to or greater than about 9.

The solvent having a higher boiling point than ethanolamine may, for example, be selected from glycerol, ethylene glycol, diethylene glycol, diethylene glycol dimethyl ether, dimethyl sulphoxide, N-methyl-2-pyrrolidone, propylene glycol and combinations of one or more thereof. The solvent may, for example, be glycerol or propylene glycol. The amount of solvent added to the fermentation composition may, for example, be any suitable amount to allow ethanolamine to be subsequently distilled from the fermentation composition. The amount of solvent added to the fermentation composition may, for example, be any suitable amount to maintain a liquid composition suitable for further processing after any water is removed from the fermentation composition, for example having a suitably low viscosity after any water is removed from the fermentation composition.

The ethanolamine products of the methods described herein may be used for any application. For example, the ethanolamine products may be used as raw materials for the synthesis of flavour compounds such as N-lactoyl ethanolamine and gluconyl ethanolamine.

The ethanolamine products may, for example, be used for the recovery and removal of acid gases (e.g. carbon dioxide, hydrogen and hydrogen sulphide) from natural, fuel and process gas. The ethanolamine products may, for example, be used for the production of monoalkanolamides for non-ionic detergents, emulsifiers and soaps. The ethanolamine products may, for example, be used for the synthesis of acelethanolamine in the manufacture of inks, paper, glues, textiles and polishes. The ethanolamine products may, for example, be used for the synthesis of phenylethanolamine in the manufacture of acetate rayon dyes and dyestuffs. The ethanolamine products may, for example, be used for the synthesis of 2-mercaptothiazole for rubber vulcanization acceleration.

### EXAMPLES

### Example 1 (Comparative Example)

The purpose of this experiment was to determine ethanolamine (MEA) yield using a solvent replacement of propylene glycol (PG) for water in a rotary evaporator. This was followed by distillation in both a wiped film still and plated column still. Yields calculated from analytical results determined whether the wiped film still process step is required to obtain pure ethanolamine, or if fractional distillation can be completed directly after solvent exchange.

### Preparation of the fermentation composition

A fermentation composition having titer of 3 g/L ethanolamine was produced by fermenting a carbon source in the presence of an ethanolamine-producing microorganism. Pure ethanolamine from Sigma Aldrich was spiked into the broth to raise the titer up to at least 35 g/L.

Any small amount of pure ethanolamine added to deionised water will raise the pH to approximately 11.6. However the broth composition is buffered as a result of the feed materials and metabolic by-products of the organism, limiting the effect of the ethanolamine addition in this case on the pH of the composition.

### Batching calculations

- Moisture content of unspiked broth = 90.51%
- Total mass of ethanolamine = 151.58g (added ethanolamine) + (3g/L*4.1791L) (produced in fermentation composition) = 164.117g (3.79%)
- Mass of solvent (propylene glycol) required = 0.24174 kg solvent per 1 kg of broth
- Total broth = 4328.0 g

### Distillation of Water in Rotary Evaporator

Propylene glycol was added to the rotary evaporator. The pressure was reduced to 50 mbar (5 kPa) and the water bath was raised to 55°C initially and then to 70°C. The boiling points of water, ethanolamine, and propylene glycol at 50 mbar (5 kPa), as determined by Antoine coefficients, are 33°C, 95°C, and 101°C, respectively.

| **Step #** | **Mass (g)** | Time | Notes |
|---|---|---|---|
| 1 | 1,010.6 | | All propylene glycol placed in the Rotavap pot |
| | 4,328.0 | | Broth prepared for feed |
| 2 | | T | Start Pressure at 50mBar |
| 3 | | T + 10 minutes | Start Temperature at 55°C |
| 4 | | 10 + 20 minutes | Start slow feed of broth into pot |
| 5 | | T + 75 minutes | Increase water bath temperature to 75°C |
| 6 | | T + 3 hours 35 minutes | Stop |

The total pot residue was 1,694.7g. The total distillate produced was 3,450.5g.

### Results

| **Sample** | **Mass (g)** | **Wt/vol% MEA** | **PPM MEA** | **Mass MEA (g)** | **% Yield** |
|---|---|---|---|---|---|
| Starting Broth | 4,328.0 | 4.34% | 43,400 | 187.83 | |
| Distillate | 3,450.5 | Not detected | 0 | 0 | 0% |
| Rotary Evaporator Concentrate | 1,694.7 | 8.71% | 87,100 | 147.6 | 79% |

The MEA was concentrated into the PG, and the water was removed as distillate without any MEA detected. The reduction in total MEA (187.83 g in broth before solvent exchange and 147.6 g in concentrate) is thought to be due to a reaction between MEA and broth impurities.

### Distillation of MEA from PG in wiped film still

The fermentation composition was fed into a wiped film still under the following conditions:

| | |
|---|---|
| Wall temperature: | 79.5°C |
| Pressure: | 8.8 mmHg |
| Feed Preheat: | 55°C |

| **Material** | **Boiling Point at 8.8 mmHg (°C)** |
|---|---|
| MEA | 69 |
| PG | 81 |
| Water | 10 |

Mass balance and analytical results from wiped film still test:

| | **Start mass (g)** | **End mass (g)** | **Mass fed to system (g)** | **Concentration of MEA (ppm)** | **Mass of MEA (g)** |
|---|---|---|---|---|---|
| **Feed Container** | 682.43 | 569.5 | 112.93 | 87.1k | 9.84 |

| | **Mass (g)** | **Concentration of MEA (ppm)** | **Mass of MEA (g)** | **% MEA (of MEA in collected receivers)** |
|---|---|---|---|---|
| **Distillate** | 19.1 | 7100 | 0.14 | 1.851 |
| **Residue** | 56.32 | 135200 | 7.61 | 98.19 |

### Result

The mass balance shows 112.93 grams material was fed to the still, while a total of 75.42 grams material was recovered in exit streams for the distillate and residue. Material will exist as holdup in the feed lines, as a thin film on the heating wall of the system and on the condensing cold finger. The propylene glycol, which has a viscosity of about 1,050 centipoise at 5°C, had condensed onto the cold finger will become chilled. Some of it remained on the cold finger in a viscous film. It often takes 15-20 minutes of feed to a still before a somewhat viscous distillate will build up enough run down the cold finger into the distillate receiver.

Only 1.8% of the MEA recovered from the material fed to the system was in the distillate, while 98.19% of the MEA was concentrated into the residue. MEA boils at a lower temperature than propylene glycol, and so the MEA was expected to be concentrated in the distillate, and have a reduced concentration in the residue (the non-volatilized portion of the feed that flows down the heated wall). In this case, the opposite result occurred. The MEA was concentrated to 13.52% in the residue, 1.55 times higher than the feed concentration, and the distillate contained PG with a concentration of MEA 12.3 times less than the feed concentration. This demonstrates that only a small portion of the ethanolamine could be volatilized. This is thought to be due to the MEA interacting with broth impurities and remaining in a non-volatile protonated state.

### Distillation of MEA in plated fractionation column

### Distillation A

751.1 g of the MEA containing PG concentrate from the rotary evaporator was added to distillation column pot flask, totalling 65.33 g MEA (751.1g * 0.0871).

The first part of the distillation was conducted at 38 mmHg. According to NIST published Antoine coefficients, the boiling points of water, MEA, and PG at 38 mmHg are 33°C, 95°C, and 110°C, respectively. The vapour temperature remained steady at approximately 33°C until thirty second time interval, at which point the vapour temperature climbed directly to over 100°C, with no plateau where the MEA is expected to distil at 95°C. Vapour temperature drops when the heat input was reduced. This is because the high differential pressure indicated column flooding. Once differential pressure dropped, the heat was re-applied and the vapour temperature again rose to approximately 106°C. The distillation was stopped due to time.

The following masses of distillate were collected, and the last cut, along with the flask residue (or bottoms), were analyzed for MEA. The first cut clearly was aqueous distillate as determined by vapour temperature. If any of the cuts were expected to be high in MEA content it would be the last cut, after all of the aqueous moisture was certainly removed during the second 'transition' cut (where the vapour temperature climbs). From the point that the water was removed, starting say, at cut two, there should be at a minimum 65 grams MEA taken as distillate. However, no MEA was detected in distillate cut five, and the concentration of MEA in the bottoms material had risen from 8.71% to 12.20%. There was no vapour temperature plateau as would have been expected at 95°C. Instead, the vapour temperature rose past the boiling point of ethanolamine. It must concluded that fraction five, containing no detectable MEA, was PG. Therefore, based on the data for both the vapour temperature stable water and PG fractions, it can be concluded that the Antoine coefficients gave boiling points at this pressure that were three to five degrees higher than the actual boiling points. This means if the MEA in the composition was able to be volatilized, even ten percent of the MEA, the vapour temperature would have levelled off in the 90°C - 92°C range. No such plateau was observed.

| **Cut** | **Mass (g)** | **MEA (%)** | **Vapour Temp (°C)** |
|---|---|---|---|
| Starting Concentrate | 751.51 | 8.71 | N/A |
| 1 | 98.79 | | 29.9-30.1 |
| 2 | 30.93 | | 30.4-103.7 |
| 3 | 4.61 | | 103.5-105.3 |
| 4 | 0.8 | | 105.3-105.6 |
| 5 | 1.49 | Not Detected | 105.6-105.8 |
| Residue | 596.04 | 12.20 | |

### Distillation B

The Bottoms material from distillation A, now at a higher concentration of MEA, was added back to the distillation column, and a lower pressure was used to attempt to distil out the ethanolamine, which was determined analytically to be retained in the bottoms material entirely. The pressure of the system was reduced to 0.2 mmHg for this distillation, which reduced the relative volatility between MEA and PG from a calculated 2.04 (at 38 mmHg) down to 1.15. This also lowered the boiling points of both materials, and so would reduce heat loss from the column to the environment, and therefore allow it to operate more efficiently. According to NIST published Antoine coefficients, the boiling points of water, MEA, and PG at 0.2 mmHg are -36°C, 22°C, and 24°C, respectively. Six distillate cuts were taken during this distillation. Each cut was analyzed, along with the remaining pot material.

Based on the data below, it can be seen that the MEA did not distil out of the broth composition concentrate, but instead PG distilled out and MEA was concentrated in the column boiling flask. Each distillate fraction was a clear liquid without colour.

| **Cut** | **Mass (g)** | **MEA (%)** |
|---|---|---|
| 1 | 10.49 | Not Detected |
| 2 | 11.54 | 0.03 |
| 3 | 9.67 | 0.03 |
| 4 | 10.29 | 0.03 |
| 5 | 12.13 | 0.03 |
| 6 | 40.89 | 0.02 |
| Residue | 356.93 | 19.74 |
| Start/Dist. A Residue | 450.56 | 12.20 |

### Results: Distillation A & B

MEA did not distil out of the PG broth composition concentrate. MEA has a lower boiling point than PG regardless of pressure, and yet clear purified PG distilled out of the concentrate while ethanolamine remained in the bottom product. This occurred while the bottoms flask is heated to a temperature well above the boiling point of both MEA and PG in their pure forms. It was hypothesized that the MEA is not distilling out of solution because it remained protonated or electronically attracted to another molecule in the concentrate, and behaved as a non-volatile salt, such as sodium chloride, would behave.

### Conclusion

It was found that pure PG was fractionally batch distilled out of the solution comprising MEA and PG, without MEA in the distillate. Thus, MEA is not volatile without a pH adjustment in PG solvent. This phenomenon of PG preferentially distilling despite having a higher boiling point than MEA was observed using both a wiped film still and a traditional batch fractionation column.

### Example 2 (Comparative Example)

The purpose of this experiment was to determine a possible MEA yield using a solvent replacement of glycerin for water in a rotary evaporator. This was followed by distillation in both a wiped film still and a glass vigreaux fractionating column. Yields calculated from analytical results determined whether the wiped film still process step is required to obtain pure ethanolamine, or if fractional distillation can be completed directly after solvent exchange.

### Preparation of the fermentation composition

A fermentation composition having titer of 8.5 g/L ethanolamine was produced by fermenting a carbon source in the presence of an ethanolamine-producing microorganism. Pure ethanolamine from Sigma Aldrich was spiked into the broth to raise the titer up to 43.2 g/L.

| **Mass (g)** | **Notes** |
|---|---|
| 2,418.2 | Broth out of fermentation at 8.5 g/L MEA |
| 87.71 | Sigma Aldrich >99% pure MEA added to fermentation broth |
| 108.26 | Total MEA in broth, now at titer 43.2 g/L, or 4.32% by mass |

### Distillation of water in rotary evaporator

775.6 g glycerin was added to the rotary evaporator. The water bath was set at 75°C, and the pressure was reduced to 50 mbar (5 kPa). The boiling point as determined by Antoine coefficients of water, ethanolamine, and glycerol at 50 mbar (5 kPa) is 33°C, 95°C, and 197°C, respectively.

All broth was fed to the system over a period of 75 minutes. Distillate was collected during this duration, and for an additional 50 minutes after all prepared broth was added to the evaporator.

| **Mass (g)** | **Notes** |
|---|---|
| 1,876.0 | Distillate recovered in receiver |
| 1,349.7 | Residue material remaining |
| 3,225.7 | Total mass out |
| 3,281.5 | Total mass in |
| 55.81 | Material not recovered (as holdup on internal system surfaces) |

Estimated composition of residue material:

| | | **Mass (g)** | | **% Total** | | **Notes** | |
|---|---|---|---|---|---|---|---|
| **Estimated water** | | 67.8 | | 5.04 | | | |
| **Broth impurities** | | 398.04 | | 29.59 | | 16.46% starting broth mass of 2418.2 g | |
| **MEA** | | 103.92 | | 7.72 | | Based on analysis of sample | |
| **Glycerin** | | 775.6 | | 57.65 | | | |
| **Total** | | 1,345.36 | | 100 | | | |
| | | | | | | | |

| | **Mass (g)** | | **Concentration of MEA (%)** | | **Mass MEA (g)** | | **% Total MEA** |
|---|---|---|---|---|---|---|---|
| **Residue** | 1876.0 | | 7.72 | | 103.92 | | 96.00 |

The distillate was not analyzed for MEA. This was because in Example 1, which used the same conditions and equipment to complete this process, with the exception of using PG instead of glycerol, MEA was not detected in the distillate. The detectable level for the analysis was 250ppm. The evaporation in this experiment was conducted in less time due to a lower amount of broth being used, and the residue retained a higher percentage of the total starting MEA mass at 96% compared to 79%. The distillate at most could have contained 0.23% MEA, or 4.34 grams out of 1,876.0 grams, which is the difference between the total MEA known to be present initially and that accounted for in the residue. This mass of MEA is more likely to have interacted with broth impurities in some manner.

### Distillation of MEA from glycerol broth composition concentrate in wiped film still

The pressure conditions initially used for this experiment in the wiped film still (WFS) was 10mmHg, however the glycerol combined with the metabolic byproducts and dissolved anions in the concentrated broth composition resulted in a material that was too viscous to process. The material in the feed line as it entered the still could be seen bubbling, and material was projected from the surface of the bubbles as they popped, generating a splattering effect that continued as the material was wiped down the heated wall of the still. This effect projected non-distilled residue mass onto the condensing cold finger of the still, and contaminated the distillate with residue material. A higher wiper rpm in addition to a reduced feed line temperature and wall temperature was tested to reduce this effect while maintaining a 10mmHg pressure, however the material was still to viscous and bubbly. In addition, it did not flow well out of the residue line once past the heated region of the wall, and the cooling residue would increase in viscosity. The formed bubbles were no longer burst and wiped down as they were formed (in the heated and wiped region), over time a foam would develop that overflowed into the distillate exit stream. For this reason the pressure was increased to 30mmHg to allow higher feed line preheat temperatures that would reduce viscosity prior to material entering the distillation chamber. The wiper speed was also increased, and heating tape was wrapped around the residue exit line to maintain a flow into the receiver and prevent material buildup in the lower region of the still. The cold trap for the system consisted of dry ice in 95% ethanol. An FMI model QVG positive displacement lab pump was used to feed material into the still, and the feed rate was controlled as a percentage of the maximum pump capability. The tables below summarize the run conditions and result for each trial. The material used for these trials is that residue produced by the rotary evaporator.

Boiling points of key materials according to NIST Antoine coefficients:

| **Pressure** | **Boiling point MEA (°C)** | **Boiling point glycerol (°C)** |
|---|---|---|
| **10 mmHg** | 72 | 170 |
| **30 mmHg** | 91 | 192 |

Summary of run conditions used in WFS:

| **Run** | **Feed Rate (%)** | **Feed Pre- heat (°C)** | **Wall Temp (°C)** | **Wipers (rpm)** | **Pressure (mmHg)** | **Feed (g)** | **Distillate (g)** | **Residue (g)** |
|---|---|---|---|---|---|---|---|---|
| **1** | 15 | 95 | 155 | 144 | 10 | X* | X* | X* |
| **2** | 8 | 70 | 120 | 288 | 10 | X* | X* | X* |
| **3** | 12 | 95- | 160 | 367 | 30 | 580.5 | 58.6 | 481.4 |

| | | | | | | | | |
|---|---|---|---|---|---|---|---|---|
| *Runs 1 & 2 had carryover into the distillate and foam carryover from the residue into the distillate. The equipment was not functioning as designed due feed properties, and the runs were stopped. | | | | | | | | |

Analytical results from WFS run 3:

| **Sample** | **[MEA] (%)** | **Total mass** | **MEA (g)** | **MEA yield (%)** |
|---|---|---|---|---|
| **Distillate** | 0.014 | 58.6 | 0.008 | 0.02 |
| **Residue** | 6.28 | 481.4 | 30.22 | 99.98 |
| **Cold Trap** | Not detected | 16.32 | X | 0.00 |

WFS Run 3 Mass Balance:

| | |
|---|---|
| 580.5 g | Total mass In, WFS run 3 |
| 556.32 g | Total mass Out. WFS run 3 |
| 24.18 g | Material remaining in system feed line and on walls as holdup |

WFS Run 3 MEA Balance:

| | |
|---|---|
| 44.70 g | MEA in feed |
| 30.30 q | MEA in exit streams |
| 14.47 g | MEA not present analytically in material exit streams |

### Result

A very small mass of MEA was detected in the distillate. The system operated at a wall temperature 69°C above the boiling point of MEA, and yet 99.98% of the MEA analyzed in exit streams remained in the distillate. The MEA did not distill out of the broth composition, but is behaving primarily as a non-volatile material.

### Distillation of MEA from glycerol using vigreaux column

This experiment was to verify in a batch distillation format that MEA was not able to be distilled from the glycerol broth composition in its present condition. This was a result of almost no MEA being distilled from the WFS process. With this test it was possible to bring the reboiler flask close to the boiling point of glycerol. 55.5 grams of glycerol concentrate were added to the bottoms flask at the start of the experiment.

### Vigreaux column:

8.5" length, 0.5" diameter
100 mL reboiler / flask
Electric 100 mL Glas-Col heating mantle
25 mL receiver
Water chilled condenser

Data summary from distillation:

| **Fraction** | **Vapour Range (°C)** | **Pot Range (°C)** | **Notes** |
|---|---|---|---|
| **1** | 23-100 | 37-107 | Clear distillate, heat power at 75% (of 110V), 6.30g collected |
| **2** | 100-100 | 107-117 | 11.29g distillate collected (spilled small amount) |
| **3** | 100-93 | 117-193 | Small swirls in receiver indicate an organic material, 5.43 grams |
| **4** | 93-87 | 193-261 | Distillate is now yellow, collected 1.17g, 80% power |
| **5** | 87-98 | 261-284 | 'smoke' became visible in system, power off, 0.77g dist. collected |

Analysis of Fractions:

| **Fraction** / **Time** | | **MEA concentration (ppm)** | **Fraction mass (g)** | **MEA mass (mg)** |
|---|---|---|---|---|
| **1** | **(1304-1508)** | 20 | 6.30 | 0.126 |
| **2** | **(1508-1555)** | X | 11.29 | X |
| **3** | **(1555-1708)** | 10 | 5.43 | 0.054 |
| **4** | **(1708-1747)** | 810 | 1.17 | 0.948 |
| **5** | **(1747-1810)** | 120 | 0.77 | 0.092 |

Fraction two was not analyzed. The vapour temperature during this fraction remained at 100°C, and was therefore expected to be entirely comprised of water. The fractions immediately prior to it and after it contained very low amounts of MEA and fraction two likely contained approximately the same level, around 10-20 ppm. The vapour temperature rose initially to 100°C, but then dropped while the pot temperature continued to rise, and faint swirls in the receiver as the distillate drops hit the liquid surface appeared to indicate some level of organic material was starting to distill. Fraction three was taken immediately at this time. Distillate rate became very slow after the drop in vapour temperature, and the last two fractions totalling 1.94 grams were collected over a period of 59 minutes. Fractions four and five were yellow-ish in color. Fractions were analyzed for ethanolamine only.

### Result of Viqreaux distillation

Total MEA determined to be present in the distillate was 1.22 milligrams, which is 0.028% of the 4.24 grams ethanolamine in the composition at the start of the experiment. If the fraction two concentration of ethanolamine was estimated at 20 ppm, then the total MEA in the distillate was 1.45 milligrams, or 0.034% of the total MEA mass.

### Conclusion of Overall Experiment

The salts in the fermentation broth remained in solution even after the water was removed and the broth composition was in a continuous glycerol phase. There was no salt precipitation during the entirety of the experiment. The salt remaining in solution affected the viscosity of the feed so that the wiped film still was limited in terms of operating at lower pressures. The MEA in the fermentation broth and in the broth concentrate composition is not volatile enough to be concentrated from the broth impurities by distillation without an adjustment in pH. This is thought to be because the MEA is acting as a non-volatile salt, and that most of the ethanolamine is remaining protonated, or that the pH of the broth composition prior to concentration and removal of water is such that the MEA is either protonated or maintaining some ionic interaction with other composition components. This is evidenced by the WFS experiment operating at a wall temperature significantly above that of the MEA boiling point at system pressure, while 99.98% of the MEA analyzed in the material after the experiment remained in the WFS residue. It was also found that MEA would not fractionally batch distill out of the glycerol concentrate. Thus, MEA is not volatile without the pH adjustment in glycerol solvent.

### Example 3

The purpose of this experiment was to determine the effect of adjusting fermentation broth pH on distillation based recovery of MEA. It examined the change in composition of distillate and residue in both a solvent exchange (glycerol for water) rotary evaporator process and upon the concentration of MEA from the glycerol composition into a distillate using a wiped film still. This Example used the same conditions and equipment as the wiped film still process from Example 2 in which 99.98% of the ethanolamine remained in the residue.

A fermentation composition having titer of 34.5 g/L ethanolamine was produced by fermenting a carbon source in the presence of an ethanolamine-producing microorganism.

### Adjustment of pH and distillation of water in rotary evaporator

| **Mass (g)** | **Notes** |
|---|---|
| 302.8 | Fermentation broth, previously centrifuged to remove biomass, pH 6.5 |
| 31.57 | 50% w/w sodium hydroxide in water added to broth with stir bar agitation |
| | Fermentation broth now at pH 12.0 |
| | Broth moisture balance result: 90.26% moisture; 9.74% solids/non-volatiles |
| 325.9 | Fermentation broth composition at pH 12.0 was added to rotary evaporator. This broth composition was analyzed and determined to have a titer of 34.5 g/L, or to contain MEA at a concentration of 3.45% by mass. |
| 99.6 | Glycerol added to rotary evaporator. The rotary evaporator was operated under the following conditions: 60rpm for bulb, 70°C water bath temperature, 50 mbar (5 kPa) pressure. The distillation operation took 47 minutes. Small salt crystals could be seen in the residue after the bulb stopped rotating. |
| 243.9 | Distillate collected from rotary evaporator. |
| 147.5 | Residue remaining in evaporator bulb. The residue was transferred to centrifuge tubes and centrifuged for five minutes at 3,000xg to drive all salt precipitate into a layer at the bottom of the tube. The material was then decanted off the salt layer into a container for feed to the wiped film still (WFS). |
| 114.2 | Broth composition concentrate decanted off of salt layer. Material carried forward as feed to WFS. |

Ethanolamine analysis of evaporator materials:

| **Sample** | **Mass (g)** | **MEA (ppm)** | **MEA (g)** | **MEA (% of total at start)** |
|---|---|---|---|---|
| **pH 12.0 Broth** | 325.9 | 34,480 | 11.24 | 100 |
| **Distillate (rotary evaporator)** | 243.9 | 770 | 0.19 | 1.69 |
| **Residue (rotary evaporator)** | 147.5 | 71,580 | 10.55 | 93.86 |

### Distillation of the ethanolamine from the glycerol broth composition using a wiped film still

The residue from the rotary evaporator process was used as feed material for the wiped film still distillation. The distillation was completed over a fifteen minute period. There was no visual splatter due to rapid bubble formation and bursting as was seen in the feed line during the WFS experiment in Example 2, nor was there any problematic foam build up in the residue exit line. The distillate was clear with a light yellow hue.

### Boiling points of key materials according to NIST Antoine coefficients

| **Pressure** | **Boiling point MEA (°C)** | **Boiling point glycerol (°C)** |
|---|---|---|
| **30 mmHg** | 91 | 192 |

Summary of run conditions used in WFS:

| **Run** | **Feed Rate (%)** | **Feed Preheat (°C)** | **Wall Temp (°C)** | **Wipers (rpm)** | **Pressure (mmHg)** | **Feed (g)** | **Distillate (g)** | **Residue (g)** |
|---|---|---|---|---|---|---|---|---|
| 1 | 12.5 | 90 | 160 | 326 | 30 | 99.55 | 11.42 | 62.59 |

Ethanolamine analysis of WFS materials

| **Sample** | **Mass (g)** | **MEA (ppm)** | **MEA (g)** | **MEA (% of total at start)** |
|---|---|---|---|---|
| **Broth composition (Feed)** | 99.55 | 71,580 | 7.13 | 100 |
| **Distillate** | 11.42 | 441,420 | 5.04 | 70.7 |
| **Residue** | 62.59 | 2,260 | 0.14 | 2.0 |

### Result

The adjustment to pH 12 resulted in a precipitation of salt from the fermentation broth after evaporation of the water produced a concentrated form of broth composition in glycerol. The distillate contained ethanolamine at 441,420 ppm, or at 44.1% by mass. The wiped film still produced ethanolamine in distillate at a concentration greater than six times that of the feed material, effectively separating the ethanolamine from the impurities in the broth composition.

### Conclusion

It is thought that the adjustment of the fermentation broth to pH 12 deprotonated the ethanolamine, removing the full '+1' charge that the molecule has at a neutral pH; a charge which caused it to act as a non-volatile salt in the fermentation composition. This was indicated by the ethanolamine concentration in the distillate of both the rotary evaporator and the wiped film still, which was elevated in comparison to earlier experiments in which a pH adjustment was not completed. In Example 1, in which fermentation broth of comparable ethanolamine levels was concentrated into propylene glycol under the same temperature and pressure conditions, there was no ethanolamine detected in the distillate. The ethanolamine in the broth of Example 1 was not volatile, despite being concentrated into a solvent of lower boiling point than glycerol, because a base sufficient to deprotonate the ethanolamine and enable distillation of the ethanolamine was not added to the broth of Example 1. During the present example, the ethanolamine was detected in the distillate at a concentration of 770 ppm. In addition, the broth used in Example 2 did not receive a base sufficient to accept a proton from ethanolamine and enable distillation of the ethanolamine prior to the wiped film still distillation of a glycerol based broth composition, and there was no concentration of ethanolamine in the distillate. In fact, the distillate contained ethanolamine at a level of only 140 ppm. Only 0.02% of the ethanolamine recovered from the system was in the distillate, while 99.98% of the recovered ethanolamine was in the residue. The glycerol broth composition used as feed material in the present example did receive addition of a base to raise the pH to greater than pH 11.6 prior to concentration into the glycerol. The wiped film still, operating under the same conditions, then generated distillate with an ethanolamine concentration of 441,420 ppm. The concentration of ethanolamine was more than six times greater than that of the feed material, and 70.7% of the ethanolamine fed to the system was recovered in the distillate, with only 2.0% of the total ethanolamine fed to the system recovered in the residue. It can therefore be concluded that a fermentation broth containing ethanolamine must be adjusted to a higher pH at which the ethanolamine is deprotonated, or the ethanolamine cannot be purified by distillation operations because it will not volatilize, even at temperatures well above its boiling point.

### Example 4

This experiment was to determine the effect of pH on the permeation of ethanolamine (MEA) in a model solution with sulfate and phosphate salts through a NFX 150-300 MWCO (molecular weight cut-off) nanofiltration membrane. If the ethanolamine were to be purified using a membrane based process, a nanofiltration would very likely be necessary to either retain impurities and allow MEA to permeate, or to retain MEA and allow permeation of impurities. MEA has a molecular weight of approximately 61 grams per mole, which is firmly in the middle of the molecular weight spectrum of many anionic and cationic salts. The MEA could potentially be separated from the majority of non-volatile broth impurities through a diafiltration, but would remain in solution with all or most of the ionic salts in the broth, which depending on MEA titer, may be present in molar concentrations equivalent or greater than the MEA. This may be the case if MEA were isolated from the majority of broth impurities using an ion exchange resin as well. The eluting liquid would almost necessarily contain an ion to displace the MEA as well as a counterion. For any of these reasons the effect of pH on the permeation of MEA through a nanofiltration membrane was of interest.

### Nanofiltration test at pH 10.3

A 254.42 g solution was prepared containing MEA, phosphate, and sodium sulfate at concentrations of 0.5 M, 0.07 M, and 0.25 M, respectively. The solution was measured to at pH 10.3 and had a moisture content of 95.77% as determined by a moisture balance. The solution was clear and all materials were fully dissolved. A dead end filtration style pressure cell designed to hold approximately 300mL volume for testing circular 90mm diameter flat sheet membranes was prepared with a NFX 150-300 MWCO manufactured by Synder Filtration. This sheet was prepared for filtration with a deionized water rinse and permeation of water through the sheet. The prepared solution was transferred to the cell, the cell was closed, and cell pressure was ramped up to 460 psi (3171 kPa) over a three minute period. An internal magnetic stirrer as activated at 250 rpm. The first drop of permeate fell from the permeate tube five minutes after pressure was applied, and the initial permeate rate was 42 drops per minute. The filtration was stopped at 385 minutes due to reduction in flux through the membrane sheet. Sample concentrations were determined by gas chromatography. A calibration curve was developed using dilutions of ethanolamine in ethanol. Each filtration test (at pH 10.3 and pH 7.0) was conducted at 25°C.

### Summary of start solutions and permeate fractions (F)

| **Sample** | **Start Solution** | **F1** | **F 2** | **F 3** | **F 4** | **F 5** | **F 6** | **Retentate** |
|---|---|---|---|---|---|---|---|---|
| **Mass (g)** | 254.42 | 24.23 | 26.77 | 21.94 | 27.11 | 25.36 | 10.37 | 120.04 |
| **MEA (ppm)** | 24,900 | 14,500 | 16,90 0 | X* | X* | X* | 23,600 | 24,700 |

| | | | | | | | | |
|---|---|---|---|---|---|---|---|---|
| X*: These fractions were not analyzed but are expected to be within the concentration range of fraction one through six. | | | | | | | | |

**Table 2: Moisture content**

| **Sample** | **Moisture content** | **Non-volatile content** |
|---|---|---|
| **Start Solution** | 95.77% | 4.23% |
| **Retentate** | 91.23% | 8.77% |

| | | |
|---|---|---|
| Nanofiltration Test at pH 7 | | |

This test was conducted with a solution of ethanolamine and phosphate salt. The solution contained ethanolamine and phosphate at 0.5 M, and 0.28 M, respectively. The test at pH 7 necessitated a specific amount of phosphoric acid based on the ethanolamine concentration, and therefore the test at pH 10.3, requiring less phosphoric acid, used sodium sulfate to achieve an equivalent concentration of divalent anions of similar mass in solution in an attempt to match any sort of concentration polarization or other effects at the membrane surface and/or pores. The conditions and equipment used in this test are otherwise the same as in the pH 10.3 test. During this experiment the permeate flux was much lower, and so only three fractions of permeate were taken as the filtration was allowed to continue overnight.

**Table 3: Summary of solutions and permeate fractions**

| **Sample** | **Start Solution** | **Fraction 1** | **Fraction 2** | **Fraction 3** | **Retentate** |
|---|---|---|---|---|---|
| **Mass (g)** | 249.0 | 25.9 | 87.29 | 25.17 | 110.64 |
| **MEA (ppm)** | ; 24,900 | 7,300 | X* | 15,500 | 46,800 |

| | | | | | |
|---|---|---|---|---|---|
| X*: This fraction was not analyzed but is expected to be within the concentration range of fraction one and three. | | | | | |

**Table 4: Moisture content**

| **Sample** | **Moisture content** | **Non-volatile content** |
|---|---|---|
| **Start Solution** | 94.69% | 5.31% |
| **Fraction 1** | 98.85% | 1.15% |
| **Fraction 2** | 97.68% | 2.32% |
| **Fraction 3** | 97.22% | 2.78% |

### Results

The analysis shows that at pH 10.3 ethanolamine is permeating through the membrane. Generating 135.78 grams of permeate took 385 minutes. Using the non-volatile content in in the starting solution, the total mass of non-volatile material that would have been in the permeate if the material had not been filtered can be determined at 5.74 grams. If all the non-volatile material had been rejected at the membrane, the retentate would have had a non-volatile content of 9.01%, although it was empirically determined to be at 8.77% with a moisture balance. Thus it can be determined that the retentate contained 97.82% of the total non-volatiles, or salts, in the solution. This is in line with the rejection expected from this membrane. The analysis of the starting solution and the retentate does not indicate concentration of ethanolamine in the cell, although the concentration in the permeate is lower than that of the starting solution, and approaches it over time. The initial lower concentration was expected, as a small volume of deionized water from the preparation of the membrane remained in the permeate tube and in the support and channels underneath the membrane.

Generating 138.36 grams of permeate at pH 7.0 took 1,767 minutes. This is 4.6 times longer than at pH 10.3. This indicates that there was more interaction with the dissolved species at the membrane surface than there was at the higher pH. In addition, the total solids of the permeate were slightly lower at this pH 7 than at pH 10.3. The concentration of ethanolamine in the first permeate fraction at pH 7 was half that of the concentration at pH 10.3, and each fraction was within 1.7 grams of the other. Despite close to the same mass of permeate passing the membrane in each test, the retentate at pH 7 contained ethanolamine at 1.9 times that of the retentate at pH 10.3.

### Conclusion

Adjusting the pH of the solution containing ethanolamine to the upper pH operating limit of the nanofiltration membrane significantly increased total flux through the membrane as well as ethanolamine concentration in the permeate. Adjusting the solution pH reduced total filtration time by eighty percent compared to the unadjusted solution. The solution at pH 7.0, representing a theoretical fermentation broth that does not receive a pH adjustment displays rejection of ethanolamine at the membrane surface almost twice that of the pH adjusted solution, concentrating the ethanolamine in the retentate. Adjusting the pH of this model solution, and very likely a fermentation broth, to deprotonate the ethanolamine and remove the full '+1' charge from the amine group in solution provided a significant advantage in this nanofiltration. Any of various possible downstream processing solutions to purifying ethanolamine that utilize nanofiltration are highly likely to benefit from a pH adjustment to give the ethanolamine a neutral overall charge, as demonstrated in this experiment, if permeation of the ethanolamine beneficial. In summary, raising the pH in this model system improved overall flux and ethanolamine flux through the membrane.

## Claims

1. A method for purifying ethanolamine from a fermentation composition, the method comprising:
adding a base to the fermentation composition; and
removing ethanolamine from the fermentation composition by distillation,
wherein addition of the base to the fermentation composition increases the pH of the fermentation composition from about 8 or less to equal to or greater than about pH 9, for example pH 10 or greater, for example pH 11 or greater.

2. The method of claim 1, wherein the base is a strong base, particularly a strong hydroxide base.

3. The method of any preceding claim, wherein the distillation is wiped film still or short path still distillation.

4. The method of any preceding claim, wherein the distillation occurs at a temperature equal to or less than about 170 °C.

5. The method of any preceding claim, wherein the distillation occurs at a pressure equal to or less than about 150 kPa.

6. The method of any preceding claim, wherein the method comprises sterilizing the fermentation composition prior to removing ethanolamine by distillation.

7. The method of any preceding claim, wherein the method comprises removing biomass and/or cells, microorganisms and/or biological agents from the fermentation composition, for example by filtration, prior to removing ethanolamine by distillation.

8. The method of claim 6 or 7, wherein the fermentation composition is sterilized and/or wherein the biomass and/or cells, microorganisms and/or biological agents are removed from the fermentation composition prior to addition of the base to the fermentation composition.

9. The method of any preceding claim, wherein the method comprises adding a solvent that has a boiling point that is higher than the boiling point of ethanolamine to the fermentation composition prior to removing ethanolamine by distillation.

10. The method of claim 9, wherein the solvent having a higher boiling point than the boiling point of ethanolamine is exchanged with water in the fermentation composition prior to removing ethanolamine by distillation.

11. The method of any preceding claim, wherein the method comprises synthesising ethanolamine by fermenting a carbon source in the presence of an ethanolamine-producing microorganism in order to provide a fermentation composition comprising ethanolamine.

12. The method of any preceding claim, wherein the method results in ethanolamine having a purity equal to or greater than about 60 %.

13. The method of any preceding claim, wherein the yield of purified ethanolamine is equal to or greater than about 60 %.

## Patentansprüche

1. Verfahren zum Reinigen von Ethanolamin aus einer Fermentationszusammensetzung, wobei das Verfahren Folgendes umfasst:
Hinzufügen einer Base zur Fermentationszusammensetzung; und
Entfernen von Ethanolamin aus der Fermentationszusammensetzung durch Destillation, wobei die Zugabe der Base zu der Fermentationszusammensetzung den pH-Wert der Fermentationszusammensetzung von etwa 8 oder weniger auf gleich oder größer als etwa pH 9 erhöht, zum Beispiel pH 10 oder höher, zum Beispiel pH 11 oder höher.

2. Verfahren nach Anspruch 1, wobei die Basis eine starke Basis, insbesondere eine starke Hydroxidbasis ist.

3. Verfahren nach einem der vorangehenden Ansprüche, wobei die Destillation eine Wischfilmdestillation oder eine Destillationsdestillation mit kurzem Weg ist.

4. Verfahren nach einem der vorhergehenden Ansprüche, wobei die Destillation bei einer Temperatur gleich oder weniger als etwa 170°C erfolgt.

5. Verfahren nach einem der vorhergehenden Ansprüche, wobei die Destillation bei einem Druck gleich oder weniger als etwa 150 kPa stattfindet.

6. Verfahren nach einem der vorhergehenden Ansprüche, wobei das Verfahren das Sterilisieren der Fermentationszusammensetzung vor dem Entfernen von Ethanolamin durch Destillation umfasst.

7. Verfahren nach einem der vorhergehenden Ansprüche, wobei das Verfahren das Entfernen von Biomasse und/oder Zellen, Mikroorganismen und/oder biologischen Mitteln aus der Fermentationszusammensetzung, beispielsweise durch Filtration, vor dem Entfernen von Ethanolamin durch Destillation umfasst.

8. Verfahren nach Anspruch 6 oder 7, wobei die Fermentationszusammensetzung sterilisiert wird und/oder wobei die Biomasse und/oder Zellen, Mikroorganismen und/oder biologische Mitteln vor der Zugabe der Base zur Fermentationszusammensetzung aus der Fermentationszusammensetzung entfernt werden.

9. Verfahren nach einem der vorhergehenden Ansprüche, wobei das Verfahren das Hinzufügen eines Lösungsmittels umfasst, das einen Siedepunkt hat, der höher ist als der Siedepunkt von Ethanolamin zur Fermentationszusammensetzung vor dem Entfernen von Ethanolamin durch Destillation.

10. Verfahren nach Anspruch 9, wobei das Lösungsmittel mit einem höheren Siedepunkt als der Siedepunkt von Ethanolamin in der Fermentationszusammensetzung mit Wasser ausgetauscht wird, bevor Ethanolamin durch Destillation entfernt wird.

11. Verfahren nach einem der vorhergehenden Ansprüche, wobei das Verfahren das Synthetisieren von Ethanolamin durch Fermentieren einer Kohlenstoffquelle in Gegenwart eines Ethanolamin-produzierenden Mikroorganismus umfasst, um eine Ethanolamin umfassende Fermentationszusammensetzung bereitzustellen.

12. Verfahren nach einem der vorhergehenden Ansprüche, wobei das Verfahren dazu führt, dass Ethanolamin eine Reinheit von mindestens etwa 60 % aufweist.

13. Verfahren nach einem der vorhergehenden Ansprüche, wobei die Ausbeute an gereinigtem Ethanolamin mindestens etwa 60 % beträgt.

## Revendications

1. Procédé de purification de l'éthanolamine à partir d'une composition de fermentation, le procédé comprenant :
l'ajout d'une base à la composition de fermentation ; et
l'élimination de l'éthanolamine de la composition de fermentation par distillation,
dans lequel l'ajout de la base à la composition de fermentation augmente le pH de la composition de fermentation d'environ 8 ou moins à une valeur égale ou supérieure à 9 ; par exemple, pH 10 ou supérieur, par exemple, pH 11 ou supérieur.

2. Procédé selon la revendication 1, dans lequel la base est une base forte, en particulier une base forte d'hydroxyde.

3. Procédé selon l'une des revendications précédentes, dans lequel la distillation est une distillation à film essuyé ou une distillation à court trajet.

4. Procédé selon l'une des revendications précédentes, dans lequel la distillation se produit à une température égale ou inférieure à 170 °C environ.

5. Procédé selon l'une des revendications précédentes, dans lequel la distillation se produit à une pression égale ou inférieure à environ 150 kPa.

6. Procédé selon l'une des revendications précédentes, dans lequel le procédé comprend la stérilisation de la composition de fermentation avant l'élimination de l'éthanolamine par distillation.

7. Procédé selon l'une des revendications précédentes, dans lequel le procédé comprend l'élimination de la biomasse et/ou des cellules, des micro-organismes et/ou des agents biologiques de la composition de fermentation, par exemple par filtration, avant l'élimination de l'éthanolamine par distillation.

8. Procédé selon la revendication 6 ou 7, dans lequel la composition de fermentation est stérilisée et/ou dans lequel la biomasse et/ou les cellules, les micro-organismes et/ou les agents biologiques sont éliminés de la composition de fermentation avant l'ajout de la base à la composition de fermentation.

9. Procédé selon l'une des revendications précédentes, dans lequel le procédé comprend l'ajout d'un solvant, dont le point d'ébullition est supérieur au point d'ébullition de l'éthanolamine, à la composition de fermentation avant l'élimination de l'éthanolamine par distillation.

10. Procédé selon la revendication 9, dans lequel le solvant ayant un point d'ébullition supérieur au point d'ébullition de l'éthanolamine est échangé avec de l'eau dans la composition de fermentation avant l'élimination de l'éthanolamine par distillation.

11. Procédé selon l'une des revendications précédentes, dans lequel le procédé comprend la synthèse de l'éthanolamine par fermentation d'une source de carbone en présence d'un micro-organisme produisant de l'éthanolamine afin de fournir une composition de fermentation comprenant de l'éthanolamine.

12. Procédé selon l'une des revendications précédentes, dans lequel le procédé aboutit à une éthanolamine d'une pureté égale ou supérieure à environ 60 %.

13. Procédé selon l'une des revendications précédentes, dans lequel le rendement en éthanolamine purifiée est égal ou supérieur à environ 60 %.
